Europäisches Patentamt

European Patent Office

Office européen des brevets

(19)

(11) Publication number : **0 294 933 B1**

# (12) EUROPEAN PATENT SPECIFICATION

(45) Date of publication of patent specification :
**11.03.92 Bulletin 92/11**

(51) Int. Cl.⁵ : **A61K 31/415,** A61K 9/20,
A61K 33/10, A61K 33/08

(21) Application number : **88304008.1**

(22) Date of filing : **04.05.88**

(54) **Pharmaceutical compositions.**

The file contains technical information
submitted after the application was filed and
not included in this specification

(30) Priority : **08.05.87 GB 8710965**

(43) Date of publication of application :
**14.12.88 Bulletin 88/50**

(45) Publication of the grant of the patent :
**11.03.92 Bulletin 92/11**

(84) Designated Contracting States :
**AT BE CH DE ES FR GB GR IT LI LU NL SE**

(56) References cited :
EP-A- 0 003 589
EP-A- 0 138 540
FR-M- 6 885
CHEMICAL ABSTRACTS, vol. 102, 1985, page
375, ref. no. 137799m; Columbus Ohio, US &
JP-A-59 193 825 (SUMITOMO CHEMICAL CO.
LTD) 02-11-1984.

(73) Proprietor : **SMITH KLINE & FRENCH
LABORATORIES LIMITED
Mundells
Welwyn Garden City Hertfordshire, AL7 1EY
(GB)**

(72) Inventor : **France Gordon
West Lodge 2 Harmer Green Lane
Digswell Hertfordshire AL6 0AD (GB)**
Inventor : **Leonard Graham Stanley
60 Hazelmere Road Marshalswick
St. Albans Hertfordshire AL4 9RN (GB)**
Inventor : **Pearmain, Kevin, Edward
24, The Green Stotfold,
Hitchin Hertfordshire (GB)**

(74) Representative : **Waters, David Martin, Dr. et al
Smith Kline & French Laboratories Ltd. Patent
Department Mundells
Welwyn Garden City Hertfordshire AL7 1EY
(GB)**

## Description

This invention relates to a solid pharmaceutical dosage form comprising cimetidine and an antacid and to a method for the preparation of such a dosage form.

Cimetidine is a histamine $H_2$-antagonist which has been described in U.K. Patent Specification 1,397,436. Cimetidine has been shown to be useful in the treatment of duodenal, gastric, recurrent and stomal ulceration, and reflux oesophagitis and in the management of patients who are at high risk from haemorrhage of the upper gastrointestinal tract.

Cimetidine and antacids are frequently co-administered (see for example the article by H. Allgayer and G. Paumgartner, Arzneim Forsch. pp.870-871, 34, No. 8 (1984) and EP-A-138540). The rationale for co-administration is that antacid brings about rapid relief from the symptoms of excess stomach acidity by neutralising the acid whereas the cimetidine brings about more sustained relief by inhibiting secretion of more acid.

However, it is well known (see Allgayer and Paumgartner, and Steinberg et al, New England J. Medicine, 1982; 307, 400-4) that when cimetidine is co-administered with antacids, particularly aluminium hydroxide and magnesium hydroxide, there is frequently a substantial reduction in the bioavailability of the cimetidine. The reason for the reduction in bioavailability is not clear, although a number of attempts to discover the mechanism responsible for the problem have been reported in the literature. Thus for example Allgayer and Paumgartner were unable to demonstrate why the decrease in bioavailability occurs although they indicated that it was not due to binding of the cimetidine by the antacid.

The benefits, particularly in terms of patient compliance with a treatment regimen, which would arise from an effective combination product containing cimetidine and an antacid, would be expected to be considerable. However, the problem of loss of bioavailability, and the lack of understanding of its cause have, up until now, precluded the development of such a product, as far as we are aware.

It has now been found, surprisingly, that the problem of the loss of bioavailability of the cimetidine can be solved by granulating at least part of the antacid separately, and in a particular manner, prior to mixing with the cimetidine.

In a first aspect, therefore, the invention provides a solid pharmaceutical dosage form comprising:

(i) cimetidine; and

(ii) antacid, wherein at least 50% of the antacid is in the form of granules comprising a freely water-soluble solid diluent, the antacid, and a rapidly swellable water-insoluble disintegrant.

In general, as the ratio of antacid to cimetidine is increased, it is desirable to increase the proportion of antacid granulated in this way. In one preferred embodiment of the invention, substantially all of the antacid is thus granulated.

The term "freely soluble" is known in the art as referring to a particular level of solubility; thus, in the U.S. Pharmacopoeia, it is defined as meaning that a substance can form a 10% solution in a solvent. Preferably the substance can form at least a 50% solution in water.

Typically the freely water-soluble solid diluent is a sugar and/or sugar alcohol.

Examples of sugars and sugar alcohols are sucrose, lactose, sorbitol, xylitol and mannitol, preferred diluents being lactose, sorbitol and sorbitol/lactose mixtures.

It is preferred that the ratio (w/w) of solid diluent to antacid is in the range 1:1 to 8:1, particularly 3:1.

Typically the rapidly swellable water-insoluble disintegrants are synthetic or semi-synthetic polymers of a type known in the art as superdisintegrants (see for example International Patent Application No. PCT/US 87/00302, published as WO 87/05804, and references cited therein). Examples of superdisintegrants include cross-linked polymeric disintegrants such as cross-linked carboxymethyl celluloses, particularly croscarmellose sodium and croscarmellose calcium, cross-linked polyvinyl pyrrolidone, and sodium starch glycolate.

Typically the disintegrant is present in an amount from 0.5% (w/w) to 8% (w/w), relative to the total weight of the granules, particularly 2% (w/w).

The antacid-containing granules are preferably formed by a dry granulation process, for example by compacting using a roller compacter or a tablet press, followed by milling the compact to give granules that have low friability. Suitably, in such a case, the mixture for granulating can contain a lubricant. Examples of lubricants are stearates such as magnesium stearate, and stearic acid.

The antacid typically is selected from aluminium hydroxide, magnesium hydroxide, magnesium carbonate, calcium carbonate and co-dried gels, for example aluminium hydroxide-magnesium carbonate co-dried gel. A particular antacid is aluminium hydroxide or a mixture of aluminium hydroxide and magnesium hydroxide.

In general a dosage form contains between 5 mEq and 30 mEq of antacid, and preferably 14 mEq.

The cimetidine will usually be present in an amount from 50 mg to 800 mg per dosage form, and typically a dosage form will contain 100 mg or 200 mg of cimetidine.

Examples of dosage forms include tablets, capsules and lozenges.

The compositions of the present invention can be in the form of chewable tablets, that is to say tablets which disintegrate readily in the mouth when chewed. With chewable tablets, the pronounced bitter taste of cimetidine means that in practice it is necessary to provide a means of masking the bitter taste. One means of masking the bitter taste is to coat the cimetidine with a coating agent in an amount effective to mask the bitter taste but which does not significantly affect the bioavailability of the cimetidine.

One such coating agent is dimethylaminoethylmethacrylate/methacrylic acid ester co-polymer which is sold under the trade name Eudragit® E. According to a co-pending patent application EP-A-290229 cimetidine can be granulated using Eudragit® E, in an amount 2 - 20% (w/w) relative to the cimetidine, as the granulating agent. By employing a Eudragit® E loading in this range, the bitter taste of cimetidine is masked but the dissolution characteristics and hence bioavailability remain acceptable.

In addition to cimetidine and antacid-containing granules, the solid dosage forms of the present invention can contain other pharmaceutical excipients. Thus, for example, where the dosage form is subject to a compression step, the dosage form can additionally contain a lubricating agent, typically stearic acid or a stearate salt and particularly magnesium stearate.

The compositions of the present invention can also contain additional sweeteners, for example aspartame, cyclamate and saccharin, and colouring and flavouring agents as known in the art.

The invention will now be illustrated in greater detail by the following Examples.

EXAMPLE 1

## 100 mg Chewable Tablet

### Ingredient

| Cimetidine Premix Granules | mg/tablet | %w/w |
|---|---|---|
| Cimetidine | 100.0 | 90.9 |
| Eudragit® E100* | 10.0 | 9.1 |

| Antacid (Al/Mg) Granules | mg/tablet | %w/w |
|---|---|---|
| Direct Compression Sorbitol | 590.0 | 34.01 |
| Direct Compression Lactose | | |
| Crystalline | 325.0 | 18.73 |
| Spray dried | 325.0 | 18.73 |
| Croscarmellose Sodium Type A | 30.0 | 1.73 |
| Dried Aluminium Hydroxide Gel** | 250.0 | 14.41 |
| Magnesium Hydroxide** | 200.0 | 11.53 |
| Magnesium Stearate | 15.0 | 0.86 |
| | 1735.0 | 100.00 |

| Tableting Mix for Compression | mg/tablet |
|---|---|
| Cimetidine Premix Granules | 110.0 |
| Antacid (Al/Mg) Granules | 1735.0 |
| Aspartame | 3.0 |
| Peppermint | 15.0 |
| Tutti Frutti | 5.0 |
| Spearmint | 5.0 |
| Lactose | 200.0 |
| Croscarmellose Sodium Type A | 30.0 |
| Magnesium Stearate | 15.0 |
| | 2118.0 |

\* Added to the cimetidine by granulation as a 40% w/v solution in methylene chloride. Solvent lost in processing.

\*\* Quantities used adjusted for the potencies of raw materials:
Standard quantity of Dried Aluminium Hydroxide gel is equivalent to 117.5 mg/tablet $Al_2O_3$ or 180 mg/tablet Aluminium Hydroxide ($Al(OH)_3$).

### Process Description

A 40% w/v solution of the Eudragit® E100 in methylene chloride is added with mixing to the cimetidine and blended until granules are formed. The resulting granules are dried and then sieved through a 16 mesh (1,2 mm) screen.

The aluminium hydroxide, magnesium hydroxide and other ingredients for the antacid granules are sieved through a 12 mesh (1.4 mm) screen and mixed together. The resulting mix is compressed on a rotary tablet press and the resulting compacts are milled using a 12 mesh (1,4 mm) screen.

The cimetidine granules, antacid granules and extragranular excipients are put into a cone blender and mixed thoroughly. The resulting mix is discharged from the blender and compressed on a suitable rotary tablet press fitted with the appropriate punches.

EXAMPLE 2

## 200 mg Chewable Tablet

### Ingredient

| Cimetidine Premix Granules | mg/tablet | %w/w |
|---|---|---|
| Cimetidine | 200.0 | 90.9 |
| Eudragit®E100* | 20.0 | 9.1 |

### Antacid (Al/Mg) Granules

| | mg/tablet | %w/w |
|---|---|---|
| Sorbitol: Direct Compression Grade | 295.0 | 34.01 |
| Lactose: Direct Compression Grade | | |
| Spray dried | 162.5 | 18.73 |
| Crystalline | 162.5 | 18.73 |
| Dried Aluminium Hydroxide Gel | 125.0 | 14.41 |
| Magnesium Hydroxide | 100.0 | 11.53 |
| Croscarmellose Sodium Type A | 15.0 | 1.73 |
| Magnesium Stearate | 7.5 | 0.86 |
| | 867.5 | 100.00 |

### Tableting Mix for Compression

| | mg/tablet | |
|---|---|---|
| Cimetidine Premix Granules | 220.0 | |
| Antacid (Al/Mg) Granules | 867.5 | |
| Dried Aluminium Hydroxide Gel | 125.0 | |
| Magnesium Hydroxide | 100.0 | |
| Sorbitol: Direct Compression Grade | 295.0 | |
| Lactose: Direct Compression Grade | | |
| Spray dried | 162.5 | |
| Crystalline | 162.5 | |
| Croscarmellose Sodium Type A | 45.0 | |
| Aspartame | 3.0 | |
| Aniseed | 20.0 | |
| Butterscotch | 20.0 | |
| Magnesium Stearate | 22.5 or | 37.5 |
| TOTAL | 2048.0 | 2063.0 |

\* Added to the cimetidine by granulation as a 40% w/v solution in methylene chloride. Solvent lost in processing.

Process Description

The cimetidine premix granules and antacid granules were prepared according to the method described in Example 1. The cimetidine granules and antacid granules were then blended with the remaining ingredients and compressed on a rotary press fitted with the appropriate tablet punches and dies. The formulations of the following Examples 3 and 4 were prepared in a similar manner.

EXAMPLE 3

## 200 mg Chewable Tablet

### Ingredient

| Cimetidine Premix Granules | mg/tablet | %w/w |
|---|---|---|
| Cimetidine | 200.0 | 90.9 |
| Eudragit® E100* | 20.0 | 9.1 |

Antacid (Al/Mg) Granules

| | mg/tablet | %w/w |
|---|---|---|
| Sorbitol: Direct Compression Grade | 590.0 | 34.01 |
| Lactose: Direct Compression Grade | | |
| Spray dried | 325.0 | 18.73 |
| Crystalline | 325.0 | 18.73 |
| Dried Aluminium Hydroxide Gel | 250.0 | 14.41 |
| Magnesium Hydroxide | 200.0 | 11.53 |
| Croscarmellose Sodium Type A[+] | 30.0 | 1.73 |
| Magnesium Stearate | 15.0 | 0.86 |
| | 1735.0 | 100.00 |

### Tableting Mix for Compression

| | mg/tablet |
|---|---|
| Cimetidine Premix Granules | 220.0 |
| Antacid (Al/Mg) Granules | 1735.0 |
| Microcrystalline Cellulose (Avicel® PH102)[+] | 200.0 |
| Aspartame | 10.0 |
| Aniseed | 20.0 |
| Butterscotch | 20.0 |
| Magnesium Stearate | 15.0 |
| TOTAL | 2220.0 |

* Added to the cimetidine by granulation as a 40% w/v solution in methylene chloride. Solvent lost in processing.

[+] Croscarmellose Sodium Type A and Avicel® PH102 can both be obtained from the FMC Corporation, Philadelphia PA.

EXAMPLE 4

### 100 mg Chewable Tablet

Ingredient

| Cimetidine Premix Granules | mg/tablet | %w/w |
|---|---|---|
| Cimetidine | 100.0 | 90.9 |
| Eudragit®E100* | 10.0 | 9.1 |

Antacid (Al/Mg) Granules

| Lactose: Direct Compression Grade | mg/tablet | %w/w |
|---|---|---|
| Spray dried | 190.0 | 29.23 |
| Crystalline | 190.0 | 29.23 |
| Dried Aluminium Hydroxide Gel | 125.0 | 19.23 |
| Magnesium Hydroxide | 100.0 | 15.38 |
| Croscarmellose Sodium Type A | 30.0 | 4.62 |
| Magnesium Stearate | 15.0 | 2.31 |
| | 650.0 | 100.00 |

Tableting Mix for Compression

| | mg/tablet |
|---|---|
| Cimetidine Premix Granules | 110.0 |
| Antacid (Al/Mg) Granules | 650.0 |
| Dried Aluminium Hydroxide Gel | 125.0 |
| Magnesium Hydroxide | 100.0 |
| Sorbitol: Direct Compression Grade | 590.0 |
| Lactose: Direct Compression Grade | |
| Spray dried | 190.0 |
| Crystalline | 190.0 |
| Croscarmellose Sodium Type A | 30.0 |
| Aspartame | 3.0 |
| Aniseed | 20.0 |
| Butterscotch | 20.0 |
| Magnesium Stearate | 15.0 |
| Sodium Saccharin | 5.0 |
| TOTAL | 2048.0 |

* Added to the cimetidine by granulation as a 40% w/v solution in methylene chloride. Solvent lost in processing.

## Claims

**Claims for the following Contracting States : AT, BE, CH, DE, FR, GB, IT, LI, LU, NL, SE**

1. A solid pharmaceutical dosage form comprising:

(i) cimetidine; and

(ii) antacid, wherein at least 50% of the antacid is in the form of granules comprising a freely water-soluble solid diluent, the antacid, and a rapidly swellable water-insoluble disintegrant.

2. A solid pharmaceutical dosage form according to claim 1 wherein substantially all of the antacid present is contained within the granules.

3. A dosage form according to claim 1 or 2 wherein the granules are dry-granulated.

4. A dosage form according to any one of claim 1 to 3 wherein the highly water-soluble solid diluent is a sugar or a sugar alcohol.

5. A dosage form according to any one of claims 1 to 4 wherein the ratio (w/w) of solid diluent to antacid is in the range from 1:1 to 8:1.

6. A dosage form according to any one of claims 1 to 5 wherein the disintegrant is a cross-linked carboxymethylcellulose.

7. A dosage form according to any one of claims 1 to 6 wherein the antacid is present in an amount from between 5 mEq and 30 mEq.

8. A chewable pharmaceutical tablet composition comprising:

(i) granules comprising cimetidine and a granulating agent wherein the granulating agent is a co-polymer of dimethylaminoethylmethacrylate and methacrylic acid ester in an amount of 10% (w/w) relative to the cimetidine; and

(ii) antacid-containing granules comprising aluminium hydroxide and magnesium hydroxide; a solid diluent which is lactose or a mixture of sorbitol and lactose, the ratio (w/w) of diluent to aluminium hydroxide/magnesium hydroxide being 3:1; and a disintegrant which is croscarmellose sodium, the disintegrant being present in an amount of 2% (w/w) relative to the total weight of the antacid-containing granules; wherein the antacid-containing granules are formed by dry granulation.

## Claims for the following Contracting States : GR, ES

1. A process for preparing a solid pharmaceutical dosage form comprising:

(i) cimetidine; and

(ii) antacid, wherein at least 50% of the antacid is in the form of granules comprising a freely water-soluble solid diluent, the antacid, and a rapidly swellable water-insoluble disintegrant;

which process comprises mixing the highly water-soluble solid diluent, antacid and rapidly swellable water-insoluble disintegrant and forming granules therefrom, and bringing the granules into association with the cimetidine and optionally any free antacid.

2. A process according to claim 1 for preparing a solid pharmaceutical dosage form wherein substantially all of the antacid present is contained within the granules.

3. A process according to claim 1 or 2 for preparing a solid pharmaceutical dosage form wherein the granules are dry-granulated.

4. A process according to any one of claims 1 to 3 for preparing a solid pharmaceutical dosage form wherein the highly water-soluble solid diluent is a sugar or a sugar alcohol.

5. A process according to any one of claims 1 to 4 for preparing a solid pharmaceutical dosage form wherein the ratio (w/w) of solid diluent to antacid is in the range from 1:1 to 8:1.

6. A process according to any one of claims 1 to 5 for preparing a solid pharmaceutical dosage form wherein the disintegrant is a cross-linked carboxymethylcellulose.

7. A process according to any one of claims 1 to 6 for preparing a solid pharmaceutical dosage form wherein the antacid is present in an amount from between 5 mEq and 30 mEq.

8. A process according to claim 1 for preparing a chewable pharmaceutical tablet composition comprising:

(i) granules comprising cimetidine and a granulating agent wherein the granulating agent is a co-polymer of dimethylaminoethylmethacrylate and methacrylic acid ester in an amount of 10% (w/w) relative to the cimetidine; and

(ii) antacid-containing granules comprising aluminium hydroxide and magnesium hydroxide; a solid diluent which is lactose or a mixture of sorbitol and lactose, the ratio (w/w) of diluent to aluminium hydroxide/magnesium hydroxide being 3:1; and a disintegrant which is croscarmellose sodium, the disintegrant being present in an amount of 2% (w/w) relative to the total weight of the antacid-containing granules; wherein the antacid-containing granules are formed by dry granulation.

**Patentansprüche**

**Patentanspruch für folgenden Vertragsstaat: AT, BE, CH, DE, FR, GB, IT, LI, LU, NL, SE**

1. Feste pharmazeutische Dosierungsform umfassend:
(i) Cimetidin und
(ii) Antacid, wobei mindestens 50 % des Antacids in Form von Körnchen vorliegt, die ein in Wasser leicht lösliches festes Verdünnungsmittel, das Antacid und ein rasch quellbares wasserunlösliches Zerfallmittel umfassen.

2. Feste pharmazeutische Dosierungsform nach Anspruch 1, wobei im wesentlichen das gesamte vorliegende Antacid in den Körnchen enthalten ist.

3. Dosierungsform nach Anspruch 1 oder 2, wobei die Körnchen trocken granuliert sind.

4. Dosierungsform nach einem der Ansprüche 1 bis 3, wobei das in Wasser sehr gut lösliche feste Verdünnungsmittel ein Zucker oder ein Zuckeralkohol ist.

5. Dosierungsform nach einem der Ansprüche 1 bis 4, wobei das Gewichtsverhältnis von festem Verdünnungsmittel zu Antacid im Bereich von 1:1 bis 8:1 liegt.

6. Dosierungsform nach einem der Ansprüche 1 bis 5, wobei das Zerfallmittel eine vernetzte Carboxymethylcellulose ist.

7. Dosierungsform nach einem der Ansprüche 1 bis 6, wobei das Antacid in einer Menge zwischen 5 mEq und 30 mEq vorliegt.

8. Arzneimittel in Form einer Kautablette, umfassend:
(i) Körnchen, umfassend Cimetidin und ein Granulierungsmittel, wobei das Granulierungsmittel ein Copolymerisat von Dimethylaminoethylmethacrylat und Methacrylsäureester in einer Menge von 10 % (Gew./Gew.) bezogen auf Cimetidin ist; und
(ii) antacidhaltige Körnchen, umfassend Aluminiumhydroxid und Magnesiumhydroxid, ein festes Verdümungsmittel, das Lactose oder ein Gemisch aus Sorbit und Lactose ist, im Verhältnis (Gew./Gew.) von Verdünnungsmittel zu Aluminiumhydroxid/Magnesiumhydroxid von 3:1, und ein Zerfallmittel, das Natriumcroscarmellose ist, wobei das Zerfallmittel in einer Menge von 2 % (Gew./Gew.), bezogen auf das Gesamtgewicht von antacidhaltigen Körnchen vorliegt; wobei die antacidhaltigen Körnchen durch Trockengranulierung hergestellt worden sind.

**Patentanspruch für folgenden Vertragsstaat: GR, ES**

1. Verfahren zur Herstellung einer festen pharmazeutischen Dosierungsform, umfassend:
(i) Cimetidin und
(ii) Antacid, wobei mindestens 50 % des Antacids in Form von Körnchen vorliegt, die ein in Wasser leicht lösliches festes Verdünnungsmittel, das Antacid und ein rasch quellbares wasserunlösliches Zerfallmittel; welches das Vermischen des in Wasser sehr gut löslichen festen Verdünnungsmittels, Antacid und eines rasch quellbaren wasserunlöslichen Zerfallmittels und die Herstellung von Körnchen davon und das Zusammenbringen der Körnchen mit dem Cimetidin und gegebenenfalls beliebigem freiem Antacid umfaßt.

2. Verfahren nach Anspruch 1 zur Herstellung einer festen pharmazeutischen Dosierungsform, wobei im wesentlichen das gesamte vorliegende Antacid in den Körnchen enthalten ist.

3. Verfahren nach Anspruch 1 oder 2, zur Herstellung einer festen pharmazeutischen Dosierungsform, wobei die Körnchen trocken granuliert sind.

4. Verfahren nach einem der Ansprüche 1 bis 3, zur Herstellung einer festen pharmazeutischen Dosierungsform, wobei das in Wasser sehr gut lösliche feste Verdünnungsmittel ein Zucker oder ein Zuckeralkohol ist.

5. Verfahren nach einem der Ansprüche 1 bis 4, zur Herstellung einer festen pharmazeutischen Dosierungsform, wobei das Gewichtsverhältnis von festem Verdünnungsmittel zu Antacid im Bereich von 1:1 bis 8:1 liegt.

6. Verfahren nach einem der Ansprüche 1 bis 5, zur Herstellung einer festen pharmazeutischen Dosierungsform, wobei das Zerfallmittel eine vernetzte Carboxymethylcellulose ist.

7. Verfahren nach einem der Ansprüche 1 bis 6, zur Herstellung einer festen pharmazeutischen Dosierungsform, wobei das Antacid in einer Menge zwischen 5 mEq und 30 mEq vorliegt.

8. Verfahren nach Anspruch 1 zur Herstellung eines Arzneimittels in Form einer Kautablette, umfassend:
(i) Körnchen, umfassend Cimetidin und ein Granulierungsmittel, wobei das Granulierungsmittel ein Copolymerisat von Dimethylaminoethylmethacrylat und Methacrylsäureester in einer Menge von 10 % (Gew./Gew.) bezogen auf Cimetidin ist; und

(ii) antacidhaltige Körnchen, umfassend Aluminiumhydroxid und Magnesiumhydroxid, ein festes Verdünnungsmittel, das Lactose oder ein Gemisch aus Sorbit und Lactose ist, im Verhältnis (Gew./Gew.) von Verdünnungsmittel zu Aluminiumhydroxid/Magnesiumhydroxid von 3:1, und ein Zerfallmittel, nämlich Natriumcroscarmellose, wobei das Zerfallmittel in einer Menge von 2 % (Gew./Gew.), bezogen auf das Gesamtgewicht von antacidhaltigen Körnchen vorliegt; wobei die antacidhaltigen Körnchen durch Trockengranulierung hergestellt worden sind.

## Revendications

**Revendications pour les Etats contractants suivants: AT, BE, CH, DE, FR, GB, IT, LI, LU, NL, SE**

1. Forme pharmaceutique solide comprenant :
(i) de la cimétidine ; et
(ii) un antiacide, où au moins 50 % de l'antiacide est sous la forme de granules comprenant un diluant solide facilement soluble dans l'eau, l'antiacide et un délitant insoluble dans l'eau pouvant gonfler rapidement.

2. Forme pharmaceutique solide selon la revendication 1, dans laquelle sensiblement tout l'antiacide présent est contenu dans les granules.

3. Forme pharmaceutique selon la revendication 1 ou 2, dans laquelle les granules sont granulés à sec.

4. Forme pharmaceutique selon l'une quelconque des revendications 1 à 3, dans laquelle le diluant solide facilement soluble dans l'eau est un sucre ou un sucre-alcool.

5. Forme pharmaceutique selon l'une quelconque des revendications 1 à 4, dans laquelle le rapport (en poids) du diluant solide à l'antiacide se situe dans l'intervalle de 1:1 à 8:1.

6. Forme pharmaceutique selon l'une quelconque des revendications 1 à 5, dans laquelle le délitant est une carboxyméthylcellulose réticulée.

7. Forme pharmaceutique selon l'une quelconque des revendications 1 à 6, dans laquelle l'antiacide est présent en une quantité comprise entre 5 méq et 30 méq.

8. Composition de comprimé pharmaceutique à croquer comprenant :
(i) des granules comprenant de la cimétidine et un agent de granulation dans lesquels l'agent de granulation est un copolymère de méthacrylate de diméthylaminoéthyle et d'un ester d'acide méthacrylique en une quantité de 10 % (en poids) par rapport à la cimétidine ; et
(ii) des granules contenant un antiacide comprenant de l'hydroxyde d'aluminium et de l'hydroxyde de magnésium ; un diluant solide qui est du lactose ou un mélange de sorbitol et de lactose, le rapport (en poids) du diluant à l'hydroxyde d'aluminium/hydroxyde de magnésium étant de 3:1 ; et un délitant qui est de la carboxyméthylcellulose sodique, le délitant étant présent en une quantité de 2 % (en poids) par rapport au poids total des granules contenant l'antiacide ; les granules contenant l'antiacide étant formés par granulation à sec.

**Revendications pour les Etats contractants suivants: GR, ES**

1. Procédé pour la préparation d'une forme pharmaceutique solide comprenant :
(i) de la cimétidine ; et
(ii) un antiacide, où au moins 50 % de l'antiacide est sous la forme de granules comprenant un diluant solide facilement soluble dans l'eau, l'antiacide et un délitant insoluble dans l'eau pouvant gonfler rapidement ; le procédé consistant à mélanger le diluant solide facilement soluble dans l'eau, l'antiacide et le délitant insoluble dans l'eau pouvant gonfler rapidement et les transformer des granules, et à mettre les granules en association avec la cimétidine et tout antiacide libre facultatif.

2. procédé selon la revendication 1 pour la préparation d'une forme pharmaceutique solide, dans lequel sensiblement tout l'antiacide présent est contenu dans les granules.

3. Procédé selon la revendication 1 ou 2 pour la préparation d'une forme pharmaceutique solide, dans laquelle les granules sont granulés à sec.

4. Procédé selon l'une quelconque des revendications 1 à 3 pour la préparation d'une forme pharmaceutique solide, dans lequel le diluant solide facilement soluble dans l'eau est un sucre ou un sucre-alcool.

5. Procédé selon l'une quelconque des revendications 1 à 4 pour la préparation d'une forme pharmaceutique solide, dans lequel le rapport (en poids) du diluant solide à l'antiacide se situe dans l'intervalle de 1:1 à 8:1.

6. Procédé selon l'une quelconque des revendications 1 à 5 pour la préparation d'une forme pharmaceutique solide, dans lequel le délitant est une carboxyméthylcellulose réticulée.

7. Procédé selon l'une quelconque des revendications 1 à 6 pour la préparation d'une forme pharmaceutique solide, dans lequel l'antiacide est présent en une quantité comprise entre 5 méq et 30 méq.

8. Procédé selon la revendication 1 pour la préparation d'une composition de comprimé pharmaceutique à croquer comprenant :

(i) des granules comprenant de la cimétidine et un agent de granulation dans lesquels l'agent de granulation est un copolymère de méthacrylate de diméthylaminoéthyle et d'un ester d'acide méthacrylique en une quantité de 10 % (en poids) par rapport à la cimétidine ; et

(ii) des granules contenant un antiacide comprenant de l'hydroxyde d'aluminium et de l'hydroxyde de magnésium ; un diluant solide qui est du lactose ou un mélange de sorbitol et de lactose, le rapport (en poids) du diluant à l'hydroxyde d'aluminium/hydroxyde de magnésium étant de 3:1 ; et un délitant qui est de la carboxyméthylcellulose sodique, le délitant étant présent en une quantité de 2 % (en poids) par rapport au poids total des granules contenant l'antiacide ; les granules contenant l'antiacide étant formés par granulation à sec.